# EUROPÄISCHE PATENTSCHRIFT

(11) **EP 0 573 999 B1**
(45) Veröffentlichungstag und Bekanntmachung des Hinweises auf die Patenterteilung: **26.08.1998**
(21) Anmeldenummer: 93109318.1
(22) Anmeldetag: 09.06.1993
(51) Int. Cl.: C07D 307/60

(54) **Verfahren zur Herstellung von Tetronsäurealkylestern**
Process for preparation of alkyltetronates
Procédé pour la préparation d'esters tétroniques

(30) Priorität: 11.06.1992 CH 1846/92
(43) Veröffentlichungstag der Anmeldung: 15.12.1993
(73) Patentinhaber: LONZA AG, CH-3945 Gampel/Wallis (CH)
(72) Erfinder: Lenzner, Joachim, Dr., Visp (Kanton Wallis) (CH)
(74) Vertreter: Weinhold, Peter, Dr.

(56) Entgegenhaltungen:
- EP-A- 0 216 324
- EP-A- 0 409 147
- DE-B- 2 845 037
- SYNTHESIS April 1992, STUTTGART Seiten 391 - 394 LAURENT DUC ET AL. 'Methyl (E)-4-Chloro-3-methoxy-2-butenoate: An Extremely Versatile Four Carbon Building Block'
- HOUBEN-WEYL 'Methoden der Organischen Chemie, Band VI/2' 1963 , GEORG THIEME VERLAG , STUTTGART

## Beschreibung

Die Erfindung betrifft ein neues Verfahren zur Herstellung von Tetronsäurealkylestern der allgemeinen Formel 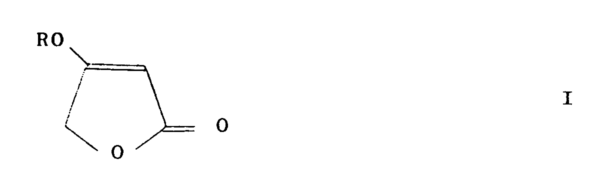 worin R eine C₁-C₆-Alkylgruppe bedeutet, ausgehend von 4-Halogen-acetessigsäurealkylestern der allgemeinen Formel 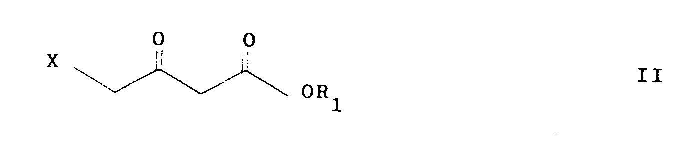 worin X Chlor oder Brom und R₁ eine C₁-C₆-Alkylgruppe bedeutet.

Tetronsäurealkylester sind z.B. wertvolle Zwischenprodukte zur Herstellung von pharmazeutischen Wirkstoffen (Pelter et al., J. Chem. Soc. Perkin Trans I, 1987, S. 717 - 742).

Bisher sind mehrere Verfahren zur Herstellung von Tetronsäurealkylestern bekannt.
Beispielsweise beschreibt die EP-A 409 147 ein Verfahren zur Herstellung von Tetronsäurealkylestern ausgehend von 4-Chlor-3-ethoxy-but-2E-ensäurealkylestern. Dabei werden die 4-Chlor-3-ethoxy-but-2E-ensäurealkylester bei Temperaturen zwischen 190 und 260°C unter Inertgasatmosphäre ohne Lösungsmittel zum entsprechenden Tetronsäurealkylester umgesetzt.

Ein grosser Nachteil dieses Verfahrens besteht darin, dass es grosstechnisch nicht durchführbar ist und Chlorethan als zu entsorgendes Abfallprodukt anfällt.

EP-A-0 216 324 beschreibt ein Verfahren zur Herstellung von 4-Halogen-3-pyrrolin-2-on-1-yl-essigsäurealkylestern über 4-Halogen-3-alkoxy-2-E-butensäurealkylester. Letztere werden durch Umsetzung von 4-Halogenacetessigsäurealkylestern mit einem Orthoameisensäuretrialkylester in saurem Medium erhalten.

Gemäß DE-A-28 45 037 kann Tetronsäuremethylester, ausgehend von Acetessigester, durch Cyclisierung eines 4-Brom-3-methoxy-but-2-encarbonsäure-niederalkylesters erhalten werden.

In Synthesis, April 1992, S. 391-394, ist die Herstellung von 4-Halogen-3-alkoxybut-2-ensäurealkylestern durch Umsetzung von 4-Halogenacetessigsäurealkylestern mit Thionylchlorid in einem Alkohol beschrieben. Es wird beschrieben, die resultierenden Butensäurealkylester thermisch, bei Temperaturen über 200°C, zu den entsprechenden Tetronsäurealkylestern zu cyclisieren.

Aufgabe der Erfindung war ein grosstechnisch gangbares und ökologisches Verfahren zur Herstellung von Tetronsäurealkylestern zur Verfügung zu stellen.

Diese Aufgabe wurde mit dem neuen Verfahren gelöst.

Erfindungsgemäss wird das Verfahren derart durchgeführt, dass man in einer ersten Stufe einen 4-Halogen-acetessigsäurealkylester der allgemeinen Formel 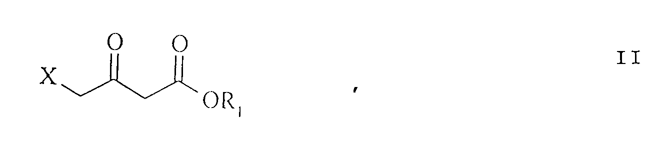 worin X und R₁ die bereits genannte Bedeutung haben, mit einem Dialkylsulfit der allgemeinen Formel

(RO)₂S=O III

worin R die genannte Bedeutung hat, in den entsprechenden intermediär gebildeten Ketalester überführt, diesen in Gegenwart einer starken Säure in einen 4-Halogen-3-alkoxy-but-2E-ensäurealkylester der allgemeinen Formel 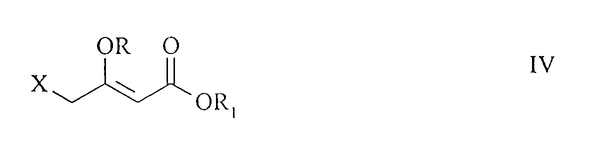 worin X, R und R₁ die genannte Bedeutung haben, überführt, diesen dann in einer zweiten Stufe mit einem Formiat und einer starken Säure, bei einer Temperatur von 110 bis 130 °C, zum Endprodukt gemäss Formel I cyclisiert.

Das Edukt des Verfahrens der 4-Halogen-acetessigsäure-C₁-C₆-alkylester kann grosstechnisch aus Diketen und dem entsprechenden Halogen über das entsprechende Säurechlorid hergestellt werden.

Geeignete zweckmässige Vertreter der 4-Halogen-acetessigsäurealkylester sind 4-Halogen-acetessigsäure-methyl, -ethyl-, -propyl-, -isopropyl- oder -butylester, worin Halogen ein Brom- oder Chloratom bedeutet.
Vorzugsweise wird 4-Chloracetessigsäureethylester angewendet.

Zweckmässig wird das Dialkylsulfit der Formel III in situ durch Umsetzung von Thionylchlorid mit dem entsprechenden aliphatischen Alkohol gebildet. Als aliphatische Alkohole können Methanol, Ethanol, Propanol, Isopropanol oder Butanol angewendet werden. Vorzugsweise wird Ethanol angewendet, wobei demgemäss als Dialkylsulfit Diethylsulfit entsteht. Zweckmässig wird der Alkohol zur in situ-Bildung des Dialkylsulfits im Überschuss eingesetzt, vorzugsweise in einer Menge von 2,5 bis 4 mol pro mol Thionylchlorid.

Bis zur Bildung des Ketalesters wird die Umsetzung in der ersten Stufe zweckmässig bei einer Temperatur von -10 bis 60°C durchgeführt.
Anschliessend wird die Umsetzung bis zum 4-Halogen-3-alkoxy-but-2E-ensäurealkylester, Formel IV, in Gegenwart einer starken Säure, bei einer zweckmässigen Temperatur von 60°C bis 120°C, durchgeführt.
Zweckmässig werden dabei die bei der Reaktion entstandenen leichter siedenden Komponenten destillativ entfernt.

Als starke Säuren sind beispielsweise Methansulfonsäure oder p-Toluolsulfonsäure geeignet.
Vorzugsweise wird als starke Säure Methansulfonsäure angewendet.
Zweckmässig wird die starke Säure in einer katalytischen Menge von 20 bis 30 mmol pro mol 4-Halogenacetessigsäurealkylester, Formel II, eingesetzt.

Nach einer üblichen Umsetzungszeit von 3 bis 6 h, kann dann der 4-Halogen-3-alkoxy-but-2E-ensäurealkylester gemäss Formel 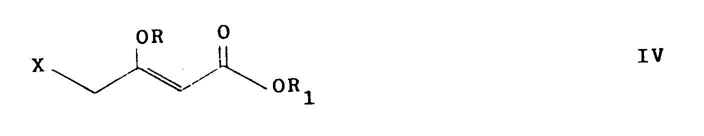 isoliert oder direkt für die zweite Stufe eingesetzt werden. Vorzugsweise wird der 4-Halogen-3-alkoxy-but-2E-ensäurealkylester direkt, ohne Isolation, für die zweite Stufe eingesetzt.

Die zweite Stufe, die Cyclisierung des 4-Halogen-3-alkoxy-but-2E-ensäurealkylesters zum Tetronsäurealkylester, wird mit einem Formiat und einer starken Säure durchgeführt.

Als starke Säuren können die gleichen, wie zuvor in der ersten Stufe beschrieben, angewendet werden.

Als Formiate können Alkali- oder Erdalkaliformiate angewendet werden. Als Alkaliformiat kann beispielsweise Natrium- oder Kaliumformiat angewendet werden. Als Erdalkaliformiat kann beispielsweise Magnesium- oder Calciumformiat Anwendung finden.

Zweckmässig werden die Formiate im Überschuss bezüglich der 4-Halogen-3-alkoxy-but-2E-ensäurealkylester, Formel IV, eingesetzt. Vorzugsweise beträgt der Überschuss 5 bis 15 Gew.% bezüglich der 4-Halogen-3-alkoxy-but-2E-ensäurealkylester.

Als Lösungsmittel können für die zweite Stufe polare aprotische Lösungsmittel angewendet werden.
Als polar aprotisches Lösungsmittel wird zweckmässig eine Mischung aus Dimethylformamid und Wasser verwendet. Vorzugsweise wird Dimethylformamid mit Wasser in einem molaren Verhältnis von Dimethylformamid zu Wasser von 1,5 bis 1,8 gemischt.

Die Umsetzung in der zweiten Stufe wird bei einer Temperatur von 110 bis 130°C, vorzugsweise von 115 bis 120°C, durchgeführt.

Nach einer üblichen Umsetzungszeit von 10 bis 15 h kann dann nach fachmännisch üblichen Methoden der Tetronsäurealkylester in guter Ausbeute isoliert werden.

### Beispiel

### Herstellung von Tetronsäureethylester

Unter Inertgasatmosphäre wurde 4-Chloracetessigsäureethylester (447,0 kg; 2,7 kmol) und abs. Ethanol (621,4 l) vorgelegt.
Bei einer Temperatur zwischen -5 und -10°C wurde anschliessend Thionylchlorid (349,2 kg; 2,94 kmole) zudosiert, so dass die Temperatur nicht über +20°C stieg. Nach Zugabe von Thionylchlorid wurde die Temperatur von 20°C auf 60°C (0,3°C/min) erhöht, die Methansulfonsäure (2,56 kg; 26 mole) zugegeben und die Temperatur von 60°C auf 120°C erhöht. Das dabei verdampfende Ethanol wurde aufgefangen. Dann wurde unter reduziertem Druck das bei der Umwandlung vom Ketalester zum 4-Chlor-3-ethoxy-2E-butensäureethylester entstehende Ethanol bei einer Temperatur von 110 bis 120°C abdestilliert.
Anschliessend wurde das Ganze auf 40°C abgekühlt und für die zweite Stufe wurden dann, unter Inertgasatmosphäre, Methansulfonsäure (2,5 kg; 26 mole), Natriumformiat (189,2kg; 2,74 kmole), Dimethylformamid (513,4 l) und Wasser (75,8 l) hinzugefügt.
Nach Erhöhung der Innentemperatur auf 120°C, wurde das Reaktionsgemisch 12 h lang gerührt, und die bei der Reaktion entstandenen leichter siedenden Komponenten destillativ entfernt. Anschliessend wurde Dimethylformamid bei 30 bis 50 mbar destillativ entfernt.

Das Reaktionsgemisch wurde mit Aceton (500 l) aufgeschlämmt, filtriert und nochmals mit Aceton nachgewaschen.
Anschliessend wurde das Aceton abdestilliert.
Die Tetronsäureethylester-enthaltende Lösung wurde auf 20°C abgekühlt.
Es wurden 328,2 kg Tetronsäureethylester (roh) mit einem Gehalt von 75% (gemäss GC) entsprechend einer Ausbeute von 75% erhalten.

## Patentansprüche

1. Verfahren zur Herstellung von Tetronsäurealkylestern der allgemeinen Formel 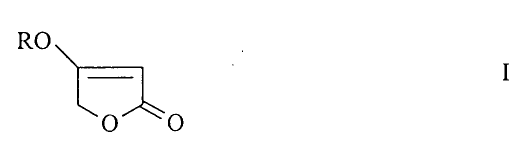 worin R eine C₁-C₆-Alkylgruppe bedeutet, dadurch gekennzeichnet, dass man in einer ersten Stufe einen 4-Halogenacetessigsäurealkylester der allgemeinen Formel 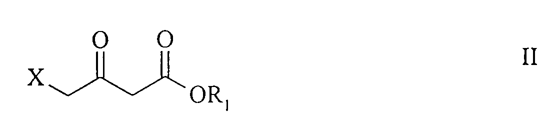 worin X Chlor oder Brom und R₁ eine C₁-C₆-Alkylgruppe bedeutet, mit einem Dialkylsulfit der allgemeinen Formel
(RO)₂S=O III
worin R die genannte Bedeutung hat, in den entsprechenden intermediär gebildeten Ketalester überführt, diesen in Gegenwart einer starken Säure in einen 4-Halogen-3-alkoxybut-2E-ensäurealkylester der allgemeinen Formel 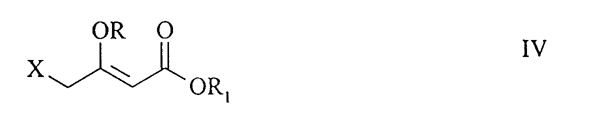 worin X, R und R₁ die genannte Bedeutung haben, überführt, diesen dann in einer zweiten Stufe mit einem Formiat und einer starken Säure, bei einer Temperatur von 110 bis 130 °C, zum Endprodukt gemäss Formel I cyclisiert.

2. Verfahren nach Patentanspruch 1, dadurch gekennzeichnet, dass man in der ersten Stufe als 4-Halogen-acetessigsäurealkylester der Formel II 4-Chloracetessigsäureethylester, anwendet.

3. Verfahren nach mindestens einem der Patentansprüche 1 bis 2, dadurch gekennzeichnet, dass man in der ersten Stufe als Dialkylsulfit der Formel III Diethylsulfit anwendet.

4. Verfahren nach Patentansprüchen 1 und 3, dadurch gekennzeichnet, dass man in der ersten Stufe das Dialkylsulfit der Formel III in situ durch Umsetzung von Thionylchlorid mit dem entsprechenden aliphatischen Alkohol herstellt.

5. Verfahren nach mindestens einem der Patentansprüche 1 bis 4, dadurch gekennzeichnet, dass man in der ersten Stufe die Umsetzung bis zum intermediär gebildeten Ketalester bei einer Temperatur von -10 bis 60°C und dann die Umsetzung bis zum 4-Halogen-3-alkoxy-but-2E-ensäurealkylester der Formel IV bei einer Temperatur von 60 bis 120°C durchführt.

6. Verfahren nach mindestens einem der Patentansprüche 1 bis 5, dadurch gekennzeichnet, dass man in der ersten und zweiten Stufe als starke Säure Methansulfonsäure oder p-Toluolsulfonsäure anwendet.

7. Verfahren nach mindestens einem der Patentansprüche 1 bis 6, dadurch gekennzeichnet, dass man in der zweiten Stufe als Formiat ein Alkali- oder Erdalkaliformiat anwendet.

8. Verfahren nach mindestens einem der Patentansprüche 1 bis 7, dadurch gekennzeichnet, dass man die Umsetzung in der zweiten Stufe in einer Mischung aus Dimethylformamid und Wasser durchführt.

9. Verfahren nach mindestens einem der Patentansprüche 1 bis 8, dadurch gekennzeichnet, dass man die Umsetzung ohne Isolation des Zwischenproduktes gemäss Formel IV durchführt.

## Claims

1. A method for preparing tetronic acid alkyl esters having the general formula 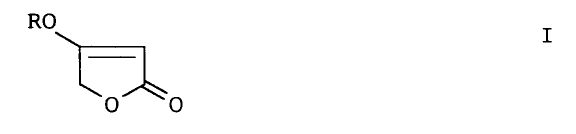 wherein R designates a C₁₋₆ alkyl group, characterised in that, in a first stage, a 4-halogen-acetoacetic acid alkyl ester having the general formula 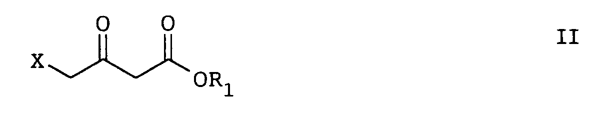 wherein X designates chlorine or bromine and R₁ designates a C₁₋₆ alkyl group, is transformed with a dialkyl sulfite having the general formula
(RO)₂S=O III
wherein R has the named meaning, into the corresponding, intermediarily formed ketal ester, and the latter is transformed in the presence of a strong acid into a 4-halogen-3-alkoxy-(E)-2-butenoic acid alkyl ester having the general formula 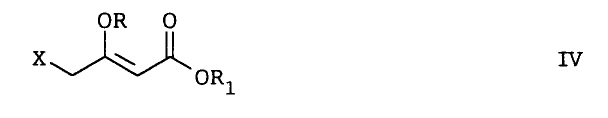 wherein X, R and R₁ have the named meanings, with the latter then being cyclised in a second stage with a formate and a strong acid, at a temperature of 110 to 130°C, into the final product according to Formula I.

2. The method according to claim 1, characterised in that, in the first stage, 4-chloroacetoacetic acid ethyl ester is utilised as said 4-halogen-acetoacetic acid alkyl ester of Formula II.

3. The method according to at least one of claims 1 to 2, characterised in that, in the first stage, diethyl sulfite is utilised as said dialkyl sulfite of Formula III.

4. The method according to claims 1 and 3, characterised in that, in the first stage, said dialkyl sulfite of Formula III is prepared *in situ* by reacting thionyl chloride with the corresponding aliphatic alcohol.

5. The method according to at least one of claims 1 to 4, characterised in that, in the first stage, reaction up to said intermediarily formed ketal ester is performed at a temperature of -10 to 60°C, and reaction up to said 4-halogen-3-alkoxy-(E)-2-butenoic acid alkyl ester of Formula IV is then performed at a temperature of 60 to 120°C.

6. The method according to at least one of claims 1 to 5, characterised in that, in the first and second stages, methanesulfonic acid or *p*-toluenesulfonic acid is utilised as a strong acid.

7. The method according to at least one of claims 1 to 6, characterised in that, in the second stage, an alkali or alkaline earth formate is utilised as a formate.

8. The method according to at least one of claims 1 to 7, characterised in that reaction in the second stage is performed in a mixture of dimethylformamide and water.

9. The method according to at least one of claims 1 to 8, characterised in that reaction is performed without isolating the intermediary product according to Formula IV.

## Revendications

1. Procédé pour la préparation d'alkylesters de l'acide tétronique de la formule générale 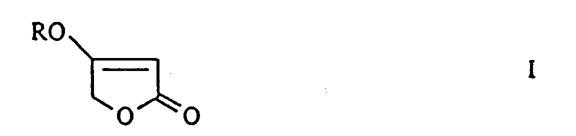 R signifiant un groupe alkyle C₁-C₆, caractérisé en ce que dans une première étape, on transforme un alkylester de l'acide 4-halogénoacétylacétique de formule générale 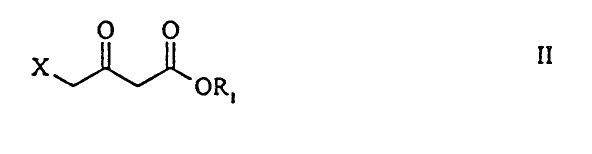 où X est le chlore ou le brome et R₁ signifie un groupe alkyle C₁-C₆ avec un dialkylsulfite de la formule générale
(RO)₂S=O III
dans laquelle R a la signification citée, en le cétalester correspondant formé de façon intermédiaire, ce en présence d'un acide fort dans un alkylester de l'acide 4-halogèn-3-alcoxybut-2E-énique de la formule générale 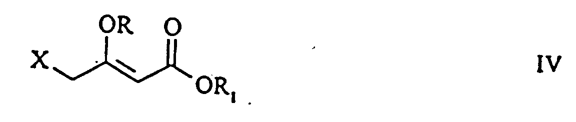 où X, R et R₁ ont la signification citée et ensuite en ce que l'on cyclise dans une deuxième étape avec un formiate et un acide fort à une température de 110 à 130°C pour obtenir le produit final selon la formule I.

2. Procédé selon la revendication 1, caractérisé en ce que dans la première étape, on utilise comme alkylester de l'acide 4-halogèn-acétique de la formule II de l'éthylester de l'acide 4-chloroacétique.

3. Procédé selon au moins l'une des revendications du brevet 1 à 2, caractérisé en ce que dans la première étape, en tant que dialkylsulfite de la formule III, on utilise du diéthylsulfite.

4. Procédé selon les revendications 1 et 3, caractérisé en ce que dans la première étape, le dialkylsulfite de la formule III est préparé in situ par réaction de chlorure de thionyle avec l'alcool aliphatique correspondant.

5. Procédé selon au moins l'une des revendications 1 à 4, caractérisé en ce que dans la première étape, on conduit la réaction jusqu'au cétalester formé de façon intermédiaire à une température de -10 à 60°C et ensuite on conduit la réaction jusqu'à l'alkylester de l'acide 4-halogèn-3-alcoxy-but-2E-énique de la formule IV à une température de 60 à 120°C.

6. Procédé selon au moins l'une des revendications 1 à 5, caractérisé en ce que dans la première et dans la deuxième étape, comme acide fort, on utilise l'acide méthanesulfonique ou l'acide p-toluènesulfonique.

7. Procédé selon au moins l'une des revendications 1 à 6, caractérisé en ce que dans la deuxième étape, comme formiate, on utilise un formiate alcalin ou un formiate alcalino-terreux.

8. Procédé selon au moins l'une des revendications 1 à 7, caractérisé en ce qu'on conduit la réaction dans la deuxième étape dans un mélange constitué de diméthylformamide et d'eau.

9. Procédé selon au moins l'une des revendications 1 à 8, caractérisé en ce que l'on conduit la réaction sans isolation du produit intermédiaire selon la formule IV.
